Europäisches Patentamt

(19) European Patent Office

Office européen des brevets

(11) EP 0 545 014 B1

(12) **EUROPEAN PATENT SPECIFICATION**

(45) Date of publication and mention
of the grant of the patent:
**06.08.1997 Bulletin 1997/32**

(51) Int. Cl.$^6$: **A61B 5/05**, A61B 5/103,
G01G 19/50

(21) Application number: **92116393.7**

(22) Date of filing: **24.09.1992**

(54) **Apparatus for measuring body fat**

Vorrichtung zur Messung von menschlichem Fett

Dispositif pour mesurer les graisses d'une personne

(84) Designated Contracting States:
**DE FR GB SE**

(30) Priority: **29.11.1991 JP 339377/91**

(43) Date of publication of application:
**09.06.1993 Bulletin 1993/23**

(73) Proprietor: **TANITA CORPORATION
Tokyo (JP)**

(72) Inventors:
 • **Sato, Tomio
  Itabashi-ku, Tokyo (JP)**
 • **Sato, Hitoshi
  Tsurugashima-shi, Saitama-ken (JP)**
 • **Oguma, Koji
  Fujisawa-shi, Kanagawa-ken (JP)**
 • **Fukuda, Yoshinori
  Itabashi-ku, Tokyo (JP)**

(74) Representative: **Hertel, Werner, Dipl.-Phys. et al
  Müller-Boré & Partner
  Patentanwälte
  Grafinger Strasse 2
  81671 München (DE)**

(56) References cited:
   GB-A- 2 215 466        US-A- 4 113 039
   US-A- 4 831 527        US-A- 4 947 862
   US-A- 4 949 727

 • PATENT ABSTRACTS OF JAPAN vol. 12, no. 008
   (P-654)12 January 1988 & JP-A-62 169 023 ( YA
   MAN LTD. )
 • The American journal of Clinical Nutrition
   41:April 1985, pp 810-817: "Assessment of fat-
   free mass using bioelectrical impedance
   measurements of human body"

**Description**

BACKGROUND OF THE INVENTION

Field of the Invention

This invention relates to an apparatus for measuring body fat to measure amount of fatty tissue in vivo.

Description of the Prior Art

It is heretofore general to decide whether or not a human being is fat from the relationship between a body weight and a height.

However it is not always unhealthy that weight is large in comparison with height, and it is important to measure fatty tissue in vivo. It is practical to measure body fat in vivo by measuring body weight. Recently various body fat measuring instruments have been sold in the market.

There has been known an underwater body weighing method for estimating body fat by calculating body density from the measured weight value under water as a method for accurately measuring body fat in vivo.

This underwater body weighing method needs a large facility, and necessitates skillful measuring technique and an effort of a human being to be measured with large influence of residual air amount in lungs to be measured.

In order to eliminate the disadvantages of the above-described underwater body weighing method, there has been proposed a method for estimating a body density by measuring a body impedance between the ends of the body and estimating the body density from the measured impedance value, height and body weight.

Since the ratio of body water occupying in fat-free tissue of body composition tissue is constant and the specific resistance of the fat-free tissue is constant, the estimation formulae for a body density = $1.1554 - 0.0841 * weight*impedance/(height)^2$ by "Segal et al" and body density = $1.1303 - 0.726*weight*impedance/(height)^2$ "Naka et al" by obtaining the inference formula as represented by body density = $A - k*weight*impedance/(height)^2$, and obtaining correlation between the constant A and the value obtained actually by an underwater body weighing method so as to decide the constants A and k, are disclosed.

However, there has been known that the impedance value is varied due to various factors such as variation in body water ratio occupying in fat-free tissue due to variation in body weight, amount of blood plasma due to variation in motion or attitude and movement of interstitial liquid. In order to stably measure the impedance value, it is necessary to consider influences of ingestion of food and water, motion, attitude, variation in body weight.

Thus, the impedance values of the same body to be measured are varied due to the causes of measuring date, time, and differences of living state before the measurement. This is too large to manage the body fat due to the variations in the measured value by the variation in the impedance if the body density is estimated by the above-described inference formula and the body fat is calculated by the formula, for causing the reliability to be reduced.

Therefore, in order to stably measure with small variation, it is necessary to maintain the body state of the patient to be measured constant by deciding a measuring date when limits in ingestions of food and water, motion are conducted and variation in body weight are small, and to measure the impedance value after the patient laid on a bed for a predetermined period of time. Thus, the measuring conditions are set.

On the other hand, there has also been proposed a method for calculating body fat from numeric values regarding a body such as an impedance between ends of the body, height, weight and sexuality by measuring the impedance by a 4-terminal Kelvin bridge living body impedance meter as a method for accurately, simply measuring the body fat in vivo as U.S. Patent Nos. 4,008,712 and 4,895,163 (Japanese Patent Laid-open No. Hei 2-60626).

In JP-A-62 169 023 there is disclosed an apparatus for measuring a fat tissue weight in a human body electrically from measuring the foot-to-foot impedance, wherein a weak AC current is run through the body via a foot electrode. The fat is calculated with a formula. The processed data include the measured impedance, sex, length, year and lengths of hands and foots.

Since the degrees of bending and opening angle of joints affect influences to the impedance values of these methods, the conditions of measuring attitude and joints of the body to be measured, laid on a bed are maintained constant so as to eliminate the erroneous causes.

Further, if an interval between a current supply terminal and a voltage detecting terminal of electrodes for detecting the impedance cannot be sufficiently obtained, it affects influence to the measured values, and hence the interval is obtained by mounting the current supply terminals on the backs of the hand and foot and the voltage detecting terminals on the tendons of the wrist and the ankle.

Accordingly, a restriction in the measuring place is large (needs a bed, etc.), and it is difficult in the apparatus to measure solely by himself to be measured.

Moreover, there has also been (see The American Journal of Clinical Nutrition 41: April 1985, pp 810-817: "Assessment of fat-free mass using bioelectrical impedance measurements of the human body"), as a method for measuring a living body impedance necessary to measure body fat as described above, a method having steps of outputting a sine wave of 50 kHz from an oscillator as a constant-voltage source, converting it to a constant current of 800 microamperes by a voltage/current converter, supplying the current through a pair of electrodes mounted on a body, outputting the voltage value of a voltage drop from a pair of electrodes mounted inside the above-described electrodes by a differential amplifier, waveform-shaping, rectifying it, DC-converting it, then, A/D-converting it as digital data, and applying it to a calculator, deciding the living body impedance Z by $Z = V/I$ from the living body current I and the terminal voltage V, and measuring the voltage V when the current I is constant, thereby deciding the living body impedance Z.

However, the constant-current source is affected to vary its output by the influence of the external environmental temperature, etc., by the degree of variation in the electrode impedance, and it is difficult to obtain the living body current I as an accurate constant current, thereby causing the measuring error of the living body impedance.

On the other hand, the living body impedance to be measured by a body fat meter needs to be accurate in a wide range of 0 to about 1 kilo-ohm. It is extremely difficult to obtain an ideal proportional relation in this range, thereby necessitating a great deal of labor for corrections of measured values.

Accordingly, an object of this invention is to provide an apparatus for measuring body fat which has a small restriction in measuring conditions and when can be simply measured without influence of the measuring condition.

Another object of this invention is to provide a practical body fat measuring apparatus which can simply measure by a measurer to be measured by himself without restriction in a measuring place.

There is provided a method for measuring body fat by the steps of measuring an impedance between ends of a body, estimating a body density from the measured impedance value, height and weight, and calculating the body fat comprising the step of estimating a body density by a calculation formula having one of a correction term for increasing the body density upon increase of the impedance with respect to only the impedance and a correction term for decreasing the body density upon increase of the weight with respect to only the weight.

According to an aspect of this inveniton, there is provided a body fat measuring apparatus for measuring body fat in a patient's body comprising a measuring station for simultaneously measuring an impedance between ends of the patient's body, a patient's height and a patient's weight, and a calculator for calculating body fat from the measured impedance value, height and weight, whereby said calculator estimates a body density by a calculation formula having one of a correction term for increasing the body density upon increase of the impedance with respect to only the impedance and a correction term for decreasing the body density upon increase of the weight with respect to only the weight.

According to still another aspect of this invention, there is provided a body fat measuring apparatus having the steps of measuring an impedance between ends of a body, and calculating body fat from numeric values of physical conditions such as the measured body impedance value, height, weight, and sexuality comprising a pair of conductive electrodes for patient's toes defined as current supply terminals at a nearest interval of 5 cm or more, and a pair of electrodes for heels defined as voltage detecting terminals on an insulating base having protrusions as heel guides in such a manner that, when the patient rides thereon at legs, the electrodes are mounted to protrude from the base symmetrically with respect to a lateral at an interval for stably standing in the upright attitude that patient's both legs are not contacted and patient's body impedance can be measured in the upright attitude.

According to another aspect, there is provided a method for measuring a living body impedance comprising the steps of inserting a plurality of reference resistors to a current path for supplying a current to a living body, measuring voltage drops of the reference resistors by momentarily switching the reference resistor group and the living body to obtain a correlation formula between the measured value by the reference resistor and the impedance, and deciding the living body's impedance by the patient's measured value and the correlation formula.

This invention will now be described in further detail with regard to preferred embodiments as illustrated in the accompanying drawing.

## BRIEF DESCRIPTION OF THE DRAWINGS

Fig. 1 is a schemataic perspective view of a body fat measuring apparatus as an embodiment of the present invention;
Fig. 2 is a plan view showing in detail a base of a body weighing meter of the apparatus of Fig. 1;
Fig. 3 is a sectional view taken along the line A - A of Fig. 2; and
Fig. 4 is a block diagram showing a circuit arrangement example for carrying out a living body impedance measuring method according to the present invention.

## DESCRIPTION OF THE PREFERRED EMBODIMENTS

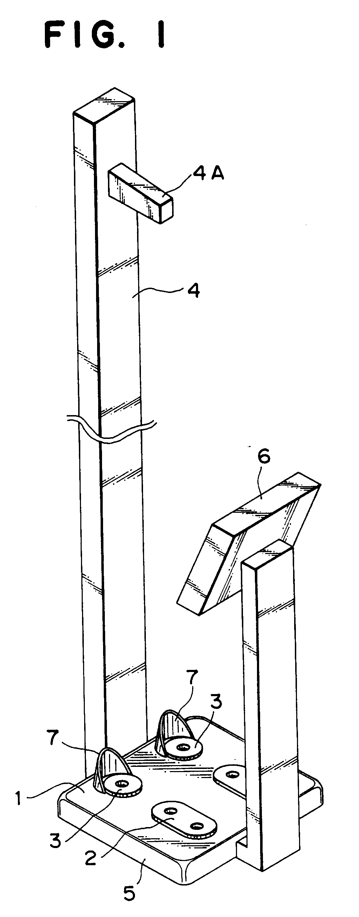

Referring to Fig. 1, a body fat measuring apparatus is shown as an embodiment of the present invention. This apparatus comprises an electronic body weighing meter 5 having a base 1 on its upper surface, an electronic height meter 4, and a display unit 6. The electronic body weighing meter 5 electronically calculates a patient's body weight value when a patient rides on the base 1. The electronic height meter 4 detects a patient's height riding on the base 1 by a detector 4A and electronically calculates the height value.

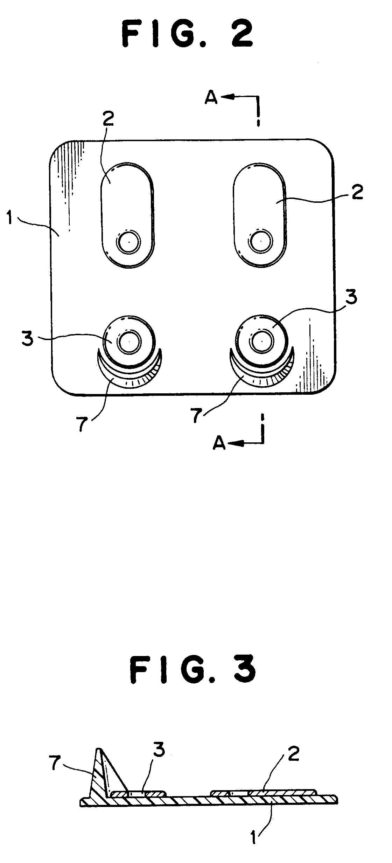

As shown in Figs. 2 and 3, the base 1 is formed of an insulating material, and a pair of conductive electrodes 3 for a patient's heels and conductive electrodes 2 for a patient's toes are mounted symmetrically with respect to a lateral at an interval in which the patient can stably stand upright. The electrodes 2 and 3 fixedly protrude from the base 1 so as to be reliably contacted with the soles of the patient's feet.

The nearest interval between the electrodes 2 and 3 is set to 5 cm or more so as to eliminate the influence of a potential distribution. The electrodes 2 are formed in an elliptical shape so as to correspond to various patient's foot size. The electrodes 2 are used as current supply electrodes, and the electrodes 3 are used as voltage detecting terminals.

Heel guides 7 are provided at the rear of the electrodes 3 so that a user can always ride at a predetermined position on the base 1 in contact with his heels.

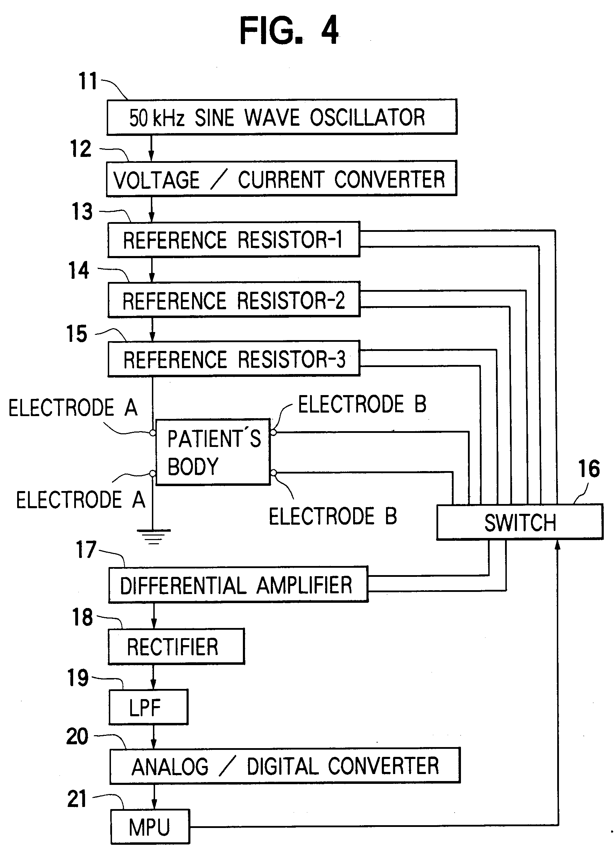

An electronic circuit for carrying out a body impedance measuring method is associated in the housing of the electronic body weighing meter 5, and an example of the electronic circuit is shown in a block diagram of Fig. 4. In Fig. 4, electrodes A correspond the electrodes 2 for the patient's toes in Figs. 1 to 3, and electrodes B correspond to the electrodes 3 for the patient's heels in Figs. 1 to 3.

The body impedance measuring electronic circuit in Fig. 4 measures a patient's body impedance as below when a patient rides on the base 1 of the apparatus in Fig. 1 in such a manner that the patient's toes and heels are contacted correspondingly with the electrodes 2 (A) and the electrodes 3 (B).

An oscillator 11 generates a sine wave of 50 kHz as a constant-voltage source, which is converted to a constant current of 800 microamperes by a voltage/current converter 12, which does not affect influence to linearity of a body impedance. The constant current is supplied from a pair of electrodes A through a plurality of reference resistor group13, 14, 15 having known resistances to cover a measuring range in average (a range necessary to measure an impedance between the ends of a patient's body as individual differences: 0 to 1 kilo-ohm).

Voltage values of voltage drops of the reference resistors 13, 14, 15 in a group, and voltage values of voltage drops of the pair of electrodes B mounted inside a living body from the electrodes A are momentarily switched by a switch 16 to be controlled by a microprocessor unit (MPU) 21 for constituting a calculator, and differences therebetween are output by a differential amplifier 17. The voltage value output by the differential amplifier 17 is rectified by a rectifier 18, waveform-shaped by a low pass filter (LPF) 19 to be DC-converted, A/D-converted by an analog/digital converter (AD) 20, and input as digital data to the microprocessor unit 21. The microprocessor unit 21 obtains a correlation formula between the measured value of the reference resistor and the impedance, and decides a living body impedance from the measured value of the patient's living body and the correlation formula.

An electronic circuit for executing a body fat measuring method is also associated in the housing of the electronic body weighing meter 5. This body fat measuring electronic circuit calculates a patient's body fat as below from the patient's weight measured by the electronic body weighing meter 5, the patient's height measured by the electronic height meter 4 and the patient's body impedance calculated by the above-described body impedance measuring electronic circuit.

An inference formula of body density (DB) by Segal from a weight (W), a height (Ht) and an impedance (Z) is represented by:

$$DB = A - k*W*Z/Ht^2 \tag{1}$$

where     A: a constant,
          k: proportional coefficient

The value proportional to the weight W and the impedance Z and inversely proportional to the square of the height is subtracted from the constant A.

However, as described above, it is known that the impedance value is varied due to various factors such as variation in a body water ratio occupying in fat-free tissue due to variation in weight, blood plasma due to variation in motion or attitude and movement of interstitial liquid in a short period. The variation in the impedances greatly affect influence to the body density in the formula (1), but the variation factor of the impedance does not originally greatly vary the body density.

In order to correct to reduce the influence of the variation in the impedances to the body density, a correction term for reversely affecting the influence of the variation in the impedances of the formula (1) to the body density, i.e., a correction term for increasing the body density if the impedance is increased is provided as a third term, and an inference formula corrected is assumed as represented by:

$$DB = A-k'*W*Z/Ht^2 + k''*Z \tag{2}$$

On the other hand, the above-described assumption is based on that the variation in the weight is small with respect to the variation in the impedance, and it can be replaced with the correction that there is variation in the weight responsive to the measured impedance. It is replaced by providing the correction term for performing the same operation as the influence of the variation in the weight in the formula (1) to the body density, i.e., the correction term for reducing the body density if the weight is increased as a third term, and a correction formula is represented by:

$$DB = A - k'^*W^*Z/HT^2 - k''^*W \qquad (3)$$

In order to stably measure the impedance, tests were conducted by a number of subjects under various conditions to obtain correlation between the above-described assumption and an underwater body weighing method, and most desirable coefficients in the formulae (2) and (3) have been decided.

The following formula could be obtained as a most suitable inference formula for estimating a patient's body density by using the impedance value between patient's both feet in an upright attitude based on the formula (2):

$$\text{Body density} = 1.1144 - 0.0976^*W^*Z/Ht^2 + 0.000084^*Z$$

where

W: body weight (kg)
Z: impedance (ohm)
Ht: height (cm)

On the other hand, the following formula could be obtained as a most suitable inference formula for estimating a patient's body density by using the impedance value between patient's both feet in an upright attitude based on the formula (3):

$$\text{Body density} = 1.1442 - 0.0626^*W^*Z/Ht^2 - 0.00044^*W$$

where

W: body weight (kg)
Z: impedance (ohm)
Ht: height (cm)

Body fat can be obtained by the following formula:

$$\text{body fat (\%)} = (4.57/\text{body density} - 4.142)^*100$$

The correlation to the underwater body weighing method of the result for calculating the body fat by the above-described inference formula is of the same degree as that by fixing the measuring conditons without correction term, the variation due to the difference of the measuring conditions is reduced to 1/2 of that without correction term, thereby obtaining the Satisfactory result.

According to the present invention, the body fat can be stably measured in the patient's upright attitude with extremely small error in the measurements by correcting the instability of the measured values due to the variation in the impedance value according to the measuring conditions.

Further, the body fat measuring apparatus according to the present invention can simply measure the user's body fat by himself merely by riding at a designated position in an upright attitude, can repeatedly measure it under the same conditions and can also measure in the upright attitude. Therefore, a restriction in the mounting position of the measuring instrument is remarkably reduced.

## Claims

1. A body fat measuring apparatus for measuring body fat in a patient's body comprising a measuring station with a base (1) on which the patient can stand, foot electrodes (2, 3), a current supplying means and an operating circuit coupled to said measuring station, **characterized in**, that the foot electrodes comprise a pair of patient heel electrodes (3) and a pair of patient toe electrodes (2), a constant current supplying means are connected between the patient toe electrodes (2) for supplying a constant current between the patient toe electrodes (2) and voltage measuring means are connected between the patient heel electrodes (3) for measuring a voltage between the patient heel electrodes (3), the patient heel electrodes (3) and the patient toe electrodes (2) being positioned on the base (1) so that bottoms of the toe and the heel of one foot of the patient and bottoms of the toe and the heel of the other foot of the patient contact each of the electrodes (2, 3) respectively, when the patient stands on the base (1), said operating circuit comprises means for calculating the impedance (Z) of the patient from the value of the constant current flowing between the patient toe electrodes (2) and the value of the voltage between the patient heel electrodes (3), means for calculating the body density (DB) of the patient from the calculated impedance (Z) of the patient, a patient's height (Ht) and a patient's weight (W) according to the following equation:

$$DB = A - k' * W * Z/Ht^2 + k'' * Z$$

where

A is a constant,
k' is a proportional coefficient,
k'' is a proportional coefficient,

and means for calculating the body fat of the patient from the calculated body density.

2. A body fat measuring apparatus for measuring body fat in a patient's body comprising a measuring station with a base (1) on which the patient can stand, foot electrodes (2, 3), a current supplying means and an operating circuit coupled to said measuring station, **characterized in**, that the foot electrodes comprise a pair of patient heel electrodes (3) and a pair of patient toe electrodes (2), a current supplying means consists of a constant current supplying means which is connected between the patient toe electrodes (2) for supplying a constant current between the patient toe electrodes (2) and voltage measuring means are connected between the patient heel electrodes (3) for measuring a voltage between the patient heel electrodes (3), the patient heel electrodes (3) and the patient toe electrodes (2) being positioned on the base (1) so that bottoms of the toe and the heel of one foot of the patient and bottoms of the toe and the heel of the other foot of the patient contact each of the electrodes (2, 3) respectively, when the patient stands on the base (1), said operating circuit comprises means for calculating the impedance (Z) of the patient from the value of the constant current flowing between the patient toe electrodes (2) and the value of the voltage between the patient heel electrodes (3), means for calculating the body density (DB) of the patient from the calculated impedance (Z) of the patient, a patient's height (Ht) and a patient's weight (W) according to the following equation:

$$DB = A - k' * W * Z/Ht^2 - k'' * W$$

where

A is a constant,
k' is a proportional coefficient,
k'' is a proportional coefficient,

and means for calculating the body fat of the patient from the calculated body density.

3. A body fat measuring apparatus as defined in claim 1 or 2, wherein the measuring station further comprises means (4, 4A) for measuring the patient's height and means (5) for measuring the patient's weight.

**Patentansprüche**

1. Körperfettmeßvorrichtung zur Messung des Körperfetts in einem Patientenkörper, umfassend eine Meßstation mit einer Basis (1), auf der ein Patient stehen kann, Fußelektroden (2, 3), eine Stromzufuhreinrichtung und eine Betriebsschaltung, die mit der Meßstation verbunden ist, **dadurch gekennzeichnet, daß** die Fußelektroden ein Paar Patientenfersenelektroden (3) und ein Paar Patientenzehenelektroden (2) umfassen, wobei eine Konstantstromzufuhreinrichtung zwischen den Patientenzehenelektroden (2) angeordnet ist, um einen Konstantstrom zwischen die Patientenzehenelektroden (2) zu liefern, und Spannungsmeßvorrichtungen zwischen den Patientenfersenelektroden (3) für das Messen einer Spannung zwischen den Patientenfersenelektroden (3) angeordnet sind, wobei die Patientenfersenelektroden (3) und die Patientenzehenelektroden (2) auf der Basis (1) so positioniert sind, daß die Unterseiten der Zehen und der Ferse eines Fußes des Patienten und die Unterseiten der Zehen und der Ferse des anderen Fußes des Patienten jeweils jede der Elektroden (2, 3) kontaktieren, wenn der Patient auf der Basis (1) steht, wobei die Betriebsschaltung umfaßt: eine Vorrichtung für das Berechnen der Impedanz (Z) des Patienten aus dem Wert des Konstantstroms, der zwischen den Patientenzehenelektroden (2) fließt, und dem Wert der Spannung zwischen den Patientenfersenelektroden (3), eine Vorrichtung zur Berechnung der Körperdichte (DB) des Patienten aus der berechneten Impedanz (Z) des Patienten, der Größe (Ht) des Patienten und dem Gewicht (W) des Patienten nach der folgenden Formel:

$$DB = A - k'^*W^*Z/Ht^2 + k''^*Z,$$

wobei

    A eine Konstante,
    k' ein Proportionalkoeffizient,
    k'' ein Proportionalkoeffizient ist,

und eine Vorrichtung zur Berechnung des Körperfetts des Patienten aus der berechneten Körperdichte.

2. Körperfettmeßvorrichtung zur Messung des Körperfetts in einem Patientenkörper, umfassend eine Meßstation mit einer Basis (1), auf der ein Patient stehen kann, Fußelektroden (2, 3), eine Stromzufuhreinrichtung und eine Betriebsschaltung, die mit der Meßstation verbunden ist, **dadurch gekennzeichnet, daß** die Fußelektroden ein Paar Patientenfersenelektroden (3) und ein Paar Patientenzehenelektroden (2) umfassen, eine Stromversorgungsvorrichtung aus einer Konstantstromversorgungsvorrichtung besteht, die zwischen den Patientenzehenelektroden (2) angeordnet ist, um einen Konstantstrom zwischen die Patientenzehenelektroden (2) zu liefern, und Spannungsmeßvorrichtungen zwischen den Patientenfersenelektroden (3) für das Messen einer Spannung zwischen den Patientenfersenelektroden (3) angeordnet sind, wobei die Patientenfersenelektroden (3) und die Patientenzehenelektroden (2) auf der Basis (1) so positioniert sind, daß die Unterseiten der Zehen und der Ferse eines Fußes des Patienten und die Unterseiten der Zehen und der Ferse des anderen Fußes des Patienten jeweils jede der Elektroden (2, 3) kontaktiert, wenn der Patient auf der Basis (1) steht, wobei die Betriebsschaltung umfaßt: eine Vorrichtung für das Berechnen der Impedanz (Z) des Patienten aus dem Wert des Konstantstroms, der zwischen den Patientenzehenelektroden (2) fließt, und dem Wert der Spannung zwischen den Patientenfersenelektroden(3), eine Vorrichtung zur Berechnung der Körperdichte (DB) des Patienten aus der berechneten Impedanz (Z) des Patienten, der Größe (Ht) des Patienten und dem Gewicht (W) des Patienten nach der folgenden Formel:

$$DB = A - k'^*W^*Z/Ht^2 - k''^*Z,$$

wobei

    A eine Konstante,
    k' ein Proportionalkoeffizient,
    k'' ein Proportionalkoeffizient ist,

und eine Vorrichtung zur Berechnung des Körperfetts des Patienten aus der berechneten Körperdichte.

3. Körperfettmeßvorrichtung nach Anspruch 1 oder 2, wobei die Meßstation weiter eine Vorrichtung (4, 4A) für das Messen der Größe des Patienten und eine Vorrichtung (5) für das Messen des Gewichts des Patienten umfaßt.

## Revendications

1. Appareil de mesure de la graisse du corps destiné à mesurer la graisse du corps dans le corps d'un patient, comprenant une station de mesure possédant une base (1) sur laquelle le patient peut se tenir debout, des électrodes de pieds (2, 3), des moyens d'alimentation d'intensité et un circuit de commande couplé à ladite station de mesure, caractérisé en ce que les électrodes de pieds comprennent une paire d'électrodes (3) de talons du patient et une paire d'électrodes (2) d'orteils du patient, des moyens d'alimentation à intensité constante sont connectés entre les électrodes (2) des orteils du patient pour appliquer une intensité constante entre les électrodes (2) d'orteils du patient et des moyens de mesure de la tension sont connectés entre les électrodes (3) de talons du patient pour mesurer une tension existant entre les électrodes (3) de talons du patient, les électrodes (3) de talons du patient et les électrodes (2) d'orteils du patient étant positionnés sur la base (1) de telle manière que les faces inférieures des orteils et du talon d'un pied du patient et les faces inférieures des orteils et du talon de l'autre pied du patient soient en contact avec chacune des électrodes (2, 3) respectivement, lorsque le patient se tient sur la base (1), ledit circuit de commande comprend des moyens servant à calculer l'impédance (Z) du patient sur la base de la valeur de l'intensité constante qui circule entre les électrodes (2) d'orteils du patient et la valeur de la tension entre les électrodes (3) de talons du patient, des moyens servant à calculer la densité du corps (DB) du patient sur la base de l'impédance calculée (Z) du patient, de la stature (Ht) du patient et du poids (W) du patient, par application de

l'équation suivante :

$$DB = A - k' * W * Z/Ht^2 + k''*Z$$

où

    A est une constante,
    k' est un coefficient de proportionnalité,
    k'' est un coefficient de proportionnalité,

et des moyens servant à calculer la graisse du corps du patient sur la base de la densité calculée du corps.

2.   Appareil de mesure de la graisse du corps destiné à mesurer la graisse du corps dans le corps d'un patient, comprenant une station de mesure possédant une base (1) sur laquelle le patient peut se tenir debout, des électrodes de pieds (2, 3), des moyens d'alimentation d'intensité et un circuit de commande couplé à ladite station de mesure, caractérisé en ce que les électrodes de pieds comprennent une paire d'électrodes (3) de talons du patient et une paire d'électrodes (2) d'orteils du patient, des moyens d'alimentation à intensité constante sont constitués par des moyens d'alimentation à intensité constante qui sont connectés entre les électrodes (2) des orteils du patient pour appliquer une intensité constante entre les électrodes (2) d'orteils du patient et des moyens de mesure de la tension sont connectés entre les électrodes (3) de talons du patient pour mesurer une tension existant entre les électrodes (3) de talons du patient, les électrodes (3) de talons du patient et les électrodes (2) d'orteils du patient étant positionnés sur la base (1) de telle manière que les faces inférieures des orteils et du talon d'un pied du patient et les faces inférieures des orteils et du talon de l'autre pied du patient soient en contact avec chacune des électrodes (2, 3) respectivement, lorsque le patient se tient sur la base (1), ledit circuit de commande comprend des moyens servant à calculer l'impédance (Z) du patient sur la base de la valeur de l'intensité constante qui circule entre les électrodes (2) d'orteils du patient et la valeur de la tension entre les électrodes (3) de talons du patient, des moyens servant à calculer la densité du corps (DB) du patient sur la base de l'impédance calculée (Z) du patient, de la stature (Ht) du patient et du poids (W) du patient, par application de l'équation suivante :

$$DB = A - k' * W * Z/Ht^2 + k''*Z$$

où

    A est une constante,
    k' est un coefficient de proportionnalité,
    k'' est un coefficient de proportionnalité,

et des moyens servant à calculer la graisse du corps du patient sur la base de la densité calculée du corps.

3.   Appareil de mesure de la graisse du corps selon les revendications 1 et 2, dans lequel la station de mesure comprend en outre des moyens (4, 4A) servant à mesurer la stature du patient et des moyens (5) servant à mesurer le poids du patient.

# FIG. 1

# FIG. 2

A

# FIG. 3

# FIG. 4

11 — 50 kHz SINE WAVE OSCILLATOR

12 — VOLTAGE / CURRENT CONVERTER

13 — REFERENCE RESISTOR-1

14 — REFERENCE RESISTOR-2

15 — REFERENCE RESISTOR-3

ELECTRODE A    ELECTRODE B

PATIENT'S BODY

ELECTRODE A    ELECTRODE B

16 — SWITCH

17 — DIFFERENTIAL AMPLIFIER

18 — RECTIFIER

19 — LPF

20 — ANALOG / DIGITAL CONVERTER

21 — MPU